# EUROPÄISCHE PATENTSCHRIFT

(11) **EP 2 869 794 B1**
(45) Veröffentlichungstag und Bekanntmachung des Hinweises auf die Patenterteilung: **09.08.2017**
(21) Anmeldenummer: 13736756.1
(22) Anmeldetag: 26.06.2013
(51) Int. Cl.: A61F 2/64, A61H 3/00, A61F 2/68, B25J 9/00, A61F 2/60, A61F 2/70, A61F 2/74, A61F 2/76

(54) **ORTHETISCHE ODER PROTHETISCHE GELENKEINRICHTUNG UND VERFAHREN ZU DESSEN STEUERUNG**
ORTHOTIC OR PROSTHETIC JOINT DEVICE, AND METHOD FOR CONTROLLING SAME
DISPOSITIF D'ARTICULATION ORTHÉTIQUE OU PROTHÉTIQUE ET PROCÉDÉ PERMETTANT DE COMMANDER LEDIT DISPOSITIF

(30) Priorität: 03.07.2012 DE 102012013141
(43) Veröffentlichungstag der Anmeldung: 13.05.2015
(73) Patentinhaber: Otto Bock HealthCare GmbH, 37115 Duderstadt (DE)
(72) Erfinder: WILL, Chistian, 37077 Göttingen (DE); PREIS, Mathias, 37339 Gernrode (DE); HARTMANN, Cornelia, 37115 Duderstadt (DE); ZARLING, Sven, 37115 Duderstadt (DE)
(74) Vertreter: Stornebel, Kai
(86) Internationale Anmeldenummer: PCT/EP2013/001868
(87) Internationale Veröffentlichungsnummer: WO 2014/005679

(56) Entgegenhaltungen:
- WO-A1-2010/088616
- WO-A2-2004/039292
- DE-A1-102008 045 113
- US-A1- 2001 029 343
- US-A1- 2007 198 098
- US-A1- 2007 233 279
- US-A1- 2008 022 672
- US-A1- 2010 305 716
- US-A1- 2010 312 363

## Beschreibung

Die Erfindung betrifft ein Verfahren zur Steuerung einer orthetischen oder prothetischen Gelenkeinrichtung.

Ziel von orthetischen oder prothetischen Einrichtungen ist es, Behinderungen im Bewegungsapparat auszugleichen. Bei Patienten, die orthetische oder prothetische Gelenkeinrichtungen an einer unteren Extremität benötigen, ist ein erhöhter Konzentrationsaufwand und ein erhöhter energetischer Aufwand notwendig, da neben den ggf. noch vorhandenen Gliedmaßen während des Gehens auch die Protheseneinrichtung oder Ortheseneinrichtung beschleunigt werden muss, also aus einer Ruhestellung in die Richtung nach vorne bewegt und vor dem Absetzen abgebremst werden muss. Um ein möglichst natürliches Gangbild zu erzeugen, sind in Protheseneinrichtungen Dämpfer vorgesehen, die vielfältige Aufgaben übernehmen. Neben einer Standphasendämpfung, die eine erhöhte Stabilität des Prothesengelenkes während der Standphase bereitstellt, ist eine Schwungphasendämpfung vorgesehen, die ein ungebremstes Schwingen in den Anschlag vermeidet. Die Bewegungsenergie wird durch den Dämpfer in Wärme umgewandelt.

Weiterhin sind aus dem Stand der Technik angetriebene Protheseneinrichtungen bekannt, die ein Verschwenken des Unterteils relativ zu dem Oberteil aktiv unterstützen. Die WO 2004/017872 A1 beschreibt eine angetriebene Prothese mit einem Prothesenkniegelenk, dem eine Dämpfereinheit zugeordnet ist. Ebenfalls ist ein Linearaktuator vorgesehen, der den Unterschenkelschaft gegenüber dem Oberteil verschwenkt. Die Energieversorgung erfolgt über einen flexiblen Batteriegurt. Der Linearmotor und der Dämpfer sind strukturell voneinander getrennt.

Die WO 2006/112774 A1 betrifft eine Kombination aus einer aktuierten und einer passiven Beinprothese sowie ein Verfahren zum Ausführen einer Bewegung mit einem entsprechenden Prothesensystem. Das Prothesensystem weist ein bewegliches Gelenk und eine Pumpe auf, die über eine Ventileinrichtung einen Hydraulikkolben in die eine oder andere Richtung bewegen kann, so dass eine Flexionsbewegung oder Extensionsbewegung des Prothesenkniegelenkes ausgeführt wird. Die Pumpe muss den gesamten Arbeitsdruck für die Bewegung aufbringen. Dies erfordert eine große Pumpenleistung, was wiederum große Pumpen benötigt, die ein großes Bauvolumen haben und entsprechend große Energiespeichereinrichtungen benötigen. Weitere orthetische oder prosthetische Kniegelenkeinrichtungen mit einem Oberteil und einem gelenkig daran angeordneten Unterteil, Befestigungseinrichtungen zum Festlegen der Gelenkeinrichtung an einem Nutzer sowie einer Antriebseinrichtung zur Beaufschlagung der Kniegelenkeinrichtung mit einem Moment, sind zum Beispiel aus US 2010/0312363, WO 2010/088616, oder US 2007/0233279 bekannt. Aufgabe der vorliegenden Erfindung ist es, ein Verfahren zur Steuerung einer orthetischen oder prothetischen Gelenkeinrichtung bereitzustellen, das trotz hoher Leistungsfähigkeit einen Energie sparenden Betrieb und damit eine lange Einsatzzeit bei einer komfortablen Steuerung ermöglicht.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung und den Figuren aufgeführt.

Die orthetische oder prothetische Gelenkeinrichtung mit einem Oberteil und einem gelenkig daran angeordneten Unterteil und Befestigungseinrichtungen zum Festlegen der Gelenkeinrichtung in einem Nutzer, mit zumindest einer Hydraulikeinheit zwischen dem Oberteil und dem Unterteil, die einen in einem Gehäuse beweglichen Kolben aufweist, der mit dem Oberteil oder dem Unterteil gekoppelt ist und dem eine Druckbereitstellungseinrichtung mit einer Pumpe und einem Druckspeicher zugeordnet ist, über die der Kolben von einer Steuereinrichtung gesteuert mit einem Druck beaufschlagt wird, sieht vor, dass der Druckspeicher mit der Pumpe in antreibender Weise koppelbar ist. Von dem Druckspeicher kann somit antreibendes Fluid zu der Pumpe geleitet werden. Die Betriebsart der Pumpe wird dabei über die anliegenden und benötigten Drücke gesteuert, die an der Hydraulikeinheit und dem Druckspeicher anliegen. Wie hoch der jeweils benötigt Druck oder Volumenstrom ist und wann welcher Druck anliegen muss, wird über Sensoren gesteuert, die Belastungen, Bewegungsgrößen und/oder Stellungen, insbesondere Winkelstellungen erfassen. Über die Pumpe kann dadurch Einfluss auf die Druckentfaltung genommen werden.

Die Pumpe kann im Generatorbetrieb betreibbar sein, so dass elektrische Energie erzeugt werden kann, wenn ein Überschuss an mechanischer oder kinetischer Energie vorhanden ist, beispielsweise beim Bergabgehen.

Eine Weiterbildung sieht vor, dass das Hydraulikfluid von dem Druckspeicher durch die Pumpe zu der Hydraulikeinheit leitbar ist. Durch die mögliche Durchleitung des Hydraulikfluids von dem Druckspeicher durch die Pumpe zu der Hydraulikeinheit ist es möglich, den Strom des Hydraulikfluids zu beeinflussen und bei einem zu hohen Druck von dem Druckspeicher durch Umschaltung auf den Generatorbetrieb eine Druckbegrenzung zu bewirken.

Sowohl die Flexionskammer als auch die Extensionskammer sind mit dem Hydraulikfluid aus dem Druckspeicher beaufschlagbar, so dass die Steuerung der Gelenkeinrichtung in allen Bewegungssituationen erfolgen kann. Eine Unterstützung der Flexion und Extension ist ebenso möglich wie das Abbremsen von Bewegungen oder das Aufbringen von Gegenkräften, die über eine Dämpfungserhöhung hinausgehen. Es hat sich herausgestellt, dass zur Steuerung orthetischer oder prothetischer Gelenkeinrichtungen relativ schnell relativ große Mengen an Hydraulikfluid bereitgestellt werden müssen, um eine zeitnahe Aktuierung der jeweiligen Komponente zu erreichen. Diejenigen Phasen, in denen eine Energiezufuhr oder eine Energieumwandlung bei einer Bewegung stattfindet, sind relativ kurz, jedoch ist eine impulsartige Beaufschlagung mit einer Druckkomponente mitunter nicht gewünscht, so dass der Impuls abgeschwächt oder die Impulscharakteristik an die jeweilige Bewegung angepasst werden muss. Dies kann durch die Durchleitung durch die Pumpe erfolgen, die als Drossel wirken kann, wobei bei einem Abbau des Druckes die Energie durch den Generatorbetrieb in elektrische Energie umwandeln lässt. Dennoch kann durch den Druckspeicher kurzfristig große Mengen an Druckfluid bereitgestellt werden, um eine präzise Steuerung und Beaufschlagung entweder der Extensionsseite oder Flexionsseite des Kolbens mit dem Druckfluid zu gewährleisten. Die Pumpe ist dem Druckspeicher nachgeschaltet, um den Druckspeicher zu unterstützen oder zu regeln.

In der Druckbereitstellungseinrichtung kann die Pumpe so mit dem Druckspeicher oder den Druckspeichern gekoppelt sein, dass über die Pumpe der Druckspeicher oder die Druckspeicher aufgefüllt werden können. Dadurch ist es möglich, dass die Pumpe nicht die volle Leistungsfähigkeit bereitstellen muss, die zur Bewegung des Kolbens notwendig ist, vielmehr kann die Pumpe den Druckspeicher oder die Druckspeicher kontinuierlich befüllen, so dass eine kleine, leichte und für einen längeren Zeitraum arbeitende Pumpe eingebaut werden kann. Darüber hinaus wird die Pumpe zum Antreiben der Hydraulikeinheit eingesetzt und kann entweder separat oder in Verbindung mit dem Druckspeicher oder den Druckspeichern zum Antreiben der Hydraulikeinheit eingesetzt werden.

Der Druckspeicher kann ausschließlich über die Pumpe mit der Hydraulikeinheit gekoppelt sein, so dass das Hydraulikfluid stets die Pumpe passieren muss, um den Kolben anzutreiben oder abzubremsen. Dadurch kann der Druck des von dem Druckspeicher abgegebenen Hydraulikfluids variiert werden, so dass entweder eine Druckerhöhung oder eine Druckminderung in der Pumpe erfolgen kann, um das von der Pumpe zu der Hydraulikeinheit geförderte Hydraulikfluid auf das benötigte Druckniveau zu bringen.

Sollte eine schlagartige Druckbeaufschlagung einer der Kammern oder beider Kammern notwendig sein, beispielsweise bei einer Sturzsituation oder einem Ausfall der Pumpe, kann eine direkte Kopplung der Kammern über eine Bypassleitung mit ggf. zwischengeschalteten Ventilen oder Drossel vorgesehen sein, um Durchleitungsverluste zu vermeiden und um das Hydraulikfluid schneller zu den Kammern transportieren zu können.

Eine Variante der Vorrichtung sieht vor, dass der Druckspeicher oder die Druckspeicher mit der Flexionskammer und/oder der Extensionskammer gekoppelt sind, so dass Hydraulikfluid aus der Extensionskammer oder Flexionskammer in den Druckspeicher gelangen, so dass bei abbremsenden Bewegungen, beispielsweise beim Treppabgehen oder beim Bergablaufen auf einer schiefen Ebene aus der entsprechenden Kammer druckbeaufschlagtes Hydraulikfluid in den Druckspeicher gepumpt wird, so dass der Druckspeicher nicht nur mit der Pumpe, sondern auch über den Hydraulikkolben selbst befüllt werden kann. Die Durchleitung zu dem Druckspeicher kann über die Pumpe erfolgen, so dass bei einem ausreichenden Druckniveau in dem Druckspeicher die Pumpe im Generatorbetrieb betrieben und zur Stromerzeugung eingesetzt werden kann.

Dem Druckspeicher kann eine Ventileinheit zugeordnet sein, über die das Hydraulikfluid dosiert und gesteuert in den Druckspeicher eingeleitet oder aus diesem herausgeleitet wird. Dadurch kann die Bewegungsenergie in Form von hydraulischem Druck in dem Druckspeicher gespeichert und kontrolliert wieder dem System zugeführt werden.

Der Gelenkeinrichtung kann ebenfalls eine Einrichtung zur Erfassung des Absolutwinkels, Gelenkwinkels, der auf das Ober- oder Unterteil wirkenden Axialkraft, des Gelenkmomentes und/oder der auf eine distale Anschlusskomponente wirkenden Momente der Umschalteinrichtung zugeordnet sein, so dass unter Berücksichtigung des Gelenkwinkels, des Absolutwinkels, der wirksamen Axialkraft oder der auf die Gelenkeinrichtung oder die Anschlussteile an die Gelenkeinrichtung wirkenden Momente eine Steuerung dahingehend erfolgen kann, ob eine Druckbeaufschlagung der Extensionskammer oder der Flexionskammer erfolgen soll.

Die Pumpe kann ein verstellbares Fördervolumen aufweisen, das an den Druck in dem Druckspeicher anpassbar ist, damit das Fördervolumen an den jeweiligen Fluidbedarf angepasst werden kann. Steigt oder sinkt der Druck in dem Druckspeicher, kann dies durch ein verringertes oder erhöhtes Fördervolumen ausgeglichen werden. Steht nur ein geringer Druck in dem Druckspeicher zur Verfügung, kann dies mit einem erhöhten Fördervolumen in Richtung Hydraulikzylinder ausgeglichen werden. Um den Druck beim Entladen aus dem Speicher noch erhöhen zu können, kann die Pumpe als eine Druckerhöhungspumpe ausgebildet sein. Es kann ebenfalls vorgesehen sein, dass die Pumpe dafür ausgebildet ist, in einem Generatorbetrieb betrieben zu werden, damit die Bewegungsenergie, die nicht unmittelbar als Druckerhöhung in dem Druckspeicher umgesetzt werden kann, genutzt wird. So kann der Druck in dem Druckspeicher wesentlich höher sein, als er im Hydraulikzylinder benötigt wird. Beim Treppenabsteigen fällt mehr Energie an als zugeführt werden müsste, so dass die kinetische Energie unmittelbar in elektrische Energie umgewandelt werden kann. Alternativ oder ergänzend kann der Druckspeicher kurzfristig mit einem sehr hohen Überdruck betrieben werden, der dann zu einem geeigneten Zeitpunkt abgebaut und ggf. ebenfalls in elektrische Energie umgewandelt wird.

Bevorzugt ist die Gelenkeinrichtung als Prothesenkniegelenk oder Orthesenkniegelenk ausgebildet, es bestehen jedoch auch andere Einsatzmöglichkeiten, beispielsweise im Rahmen eines Knöchelgelenkes oder eines Fußgelenkes, eines Hüftgelenkes oder im Rahmen von Prothesen oder Orthesen an oberen Extremitäten.

Die Hydraulikeinheit kann als Rotationshydraulik oder als Kolben-Hydraulik mit einer linearen Verlagerungsbewegung ausgebildet sein.

Eine Weiterbildung der Vorrichtung sieht vor, dass mehrere Druckspeicher vorhanden sind, die unterschiedliche Druckvolumina und/oder Druckniveaus bereitstellen. Unterschiedliche Gehsituationen benötigen unterschiedliche Unterstützungen. Beispielsweise wird beim Gehen in der Ebene in der Regel nur ein geringer Impuls oder eine geringe Unterstützung bei einer Extension oder Flexion benötigt, während beim Treppaufgehen ein relativ großes Moment zur Extensionsunterstützung bereitgestellt werden muss, um eine wirksame Unterstützung zu erzielen. Durch die Anordnung mehrerer unterschiedlicher Druckspeicher kann der jeweils günstigste Druckspeicher für die vorliegende Gehsituation ausgewählt werden, so dass weniger Energieverluste auftreten und die Befüllung der Druckspeicher schneller erfolgen kann, als wenn immer der Maximaldruck und das Maximalvolumen abgerufen werden würden.

Das erfindungsgemäße Verfahren zur Steuerung einer orthetischen oder prothetischen Kniegelenkeinrichtung mit einem Oberteil und einem gelenkig daran angeordneten Unterteil und Befestigungseinrichtungen zum Festlegen der Gelenkeinrichtung an einem Nutzer, sowie mit zumindest einem Antrieb sieht vor, dass vor der Einbeugung die Kniegelenkeinrichtung mit einem unterstützenden Beugemoment beaufschlagt wird, das unterhalb des Niveaus liegt, das zu einer Einbeugung führt. Um nicht über einen langen Zeitraum ein Beugemoment unterhalb des Niveaus einer Einbeugung bereitzuhalten, wenn im normalen Gangzyklus keine Einbeugung zu erwarten ist, also während der überwiegenden Zeit der Standphase, ist erfindungsgemäß vorgesehen, dass das Beugemoment nur in einem begrenzten Zeitraum unmittelbar vor der Einbeugung bereitgestellt und aufgebracht wird, der zwischen 5% und 40%, insbesondere zwischen 5% und 20% der Dauer eines Gangzyklusses beträgt.

Es hat sich überraschend gezeigt, dass eine erhebliche Erleichterung für einen Patienten erzielt werden kann, wenn vor dem eigentlichen Einbeugen der Kniegelenkeinrichtung ein die Einbeugung unterstützendes Moment aufgebracht wird, das jedoch so gering ist, dass noch keine Einbeugung stattfindet. Das Gelenk wird somit nicht aktiv eingebeugt, vielmehr wird das Momentenniveau oder Kraftniveau verringert, das notwendig ist, um eine Einbeugung zu erreichen. Die Kontrolle über die Einbeugung verbleibt vollständig bei dem Patienten oder dem Nutzer der orthetischen oder prothetischen Einrichtung. Die Einbeugung wird durch den Patienten bewirkt, wenn das mit der Prothese oder Orthese versorgte Bein bewegt wird. Es liegt kein rein passives Gelenk vor, das allein durch die Betätigung des Nutzers betrieben wird, vielmehr ist eine semiaktive Einrichtung erzielt, die das auslösende Moment bei dem Patienten belässt, die Auslösung selbst und eine Einbeugung erleichtert, indem das für die Einbeugung aufzubringende Moment durch ein Vorspannmoment verringert wird und bei beginnender Beugung das Moment eine definierte Zeit aufrecht erhält. Das Moment wird in die Beugung mitgenommen.

Gleiches gilt auch für ein Streckmoment, das vor dem Einleiten einer Streckbewegung oder Extensionsbewegung aufgebracht wird. Beispielsweise bei einem Treppaufgehen wird bei aktiven Kniegelenkeinrichtungen mit hohem Energieaufwand eine Anhebung des Patienten bewirkt. Es hat sich herausgestellt, dass eine erhebliche Erleichterung für einen Patienten bereits bei einer unterstützenden Aufbringung von Streckmomenten erzielt wird, wobei das Niveau des Streckmomentes unterhalb des Niveaus ist, das benötigt wird, um ein vollständiges Anheben eines Patienten auf ein höheres Niveau oder eine Bewegungsumkehr in der Schwungphase zu erreichen.

Eine Weiterbildung des Verfahrens sieht vor, dass das Beugemoment, das durch einen Druckspeicher aufgebracht wird, verringert wird, wenn die Kniegelenkeinrichtung eingebeugt oder bevor die Kniegelenkeinrichtung eingebeugt wird, so dass verhindert wird, dass nach dem anfänglichen Einbeugen und im fortgeschrittenen Gangzyklus beim Abheben des Vorderfußes von dem Boden der Unterschenkel oder die Unterschenkelkomponente ungewollt weit eingebeugt wird, so dass sich ein unnatürliches Gangbild ergeben würde. Durch das Verringern des Beugemomentes unmittelbar vor oder nach dem Einbeugen der Gelenkeinrichtung wird gewährleistet, dass das Einbeugen selbst erleichtert wird, eine aktive Flexionsunterstützung durch den Druckspeicher während der Einbeugung, beispielsweise während der Schwungphase, findet nicht statt. Auch ist es möglich, die Verringerung des Beugemomentes unmittelbar vor der Einbeugung der Kniegelenkeinrichtung zu verringern. Statt einer Verringerung auf Null kann das unterstützende Beugemoment weiterhin vorhanden sein, jedoch auch einem verringerten Niveau gegenüber dem maximalen unterstützenden Beugemoment. Ob das Kniegelenk eingebeugt wird, kann über einen Sensor ermittelt werden. Ebenfalls kann vorgesehen sein, dass erst ab einer gewissen Einbeugung des Beugemoment reduziert oder abgeschaltet wird. So ist es beispielsweise möglich, das Beugemoment erst bei einer Einbeugung beispielsweise von 4° abzuschalten oder über den zunehmenden Beugewinkel das unterstützende Beugemoment zu reduzieren. Die Reduzierung kann kontinuierlich oder diskontinuierlich erfolgen. Es kann vorgesehen sein, dass die Reduzierung beispielsweise von Beginn der Einbeugung bis zu einem vorbestimmten Kniewinkel, beispielsweise von 5° oder 6°, bis auf 0 reduziert wird.

Eine Weiterbildung der Erfindung sieht vor, dass in Abhängigkeit von der Gehsituation ein unterschiedlich hohes Beugemoment oder Streckmoment bereitgestellt wird. Insbesondere beim normalen Gehen in der Ebene kommt es zu einem wiederholten, gleichmäßigen Bewegungsablauf, der markante Kenngrößen aufweist, anhand derer das Vorliegen oder Nichtvorliegen einer Gehsituation detektiert werden können. Das Gehen in der Ebene lässt sich beispielsweise anhand eines markanten Kniewinkelverlaufes detektieren. Wird eine solche Gehsituation detektiert, kann ein daran angepasstes Beugemoment bereitgestellt werden. Wird beispielsweise ein Bergaufgehen oder Treppensteigen detektiert, wird statt oder ergänzend zu einem Beugemoment vor Einleitung der Schwungphase ein Extensionsmoment aufgebracht, das unterhalb des Niveaus der eigenständigen Anhebung des Patienten liegt, um dem Patienten eine Erleichterung zu verschaffen.

Weiterhin ist es möglich und vorgesehen, dass in Abhängigkeit von dem Prothesenaufbau ein unterschiedliches Beugemoment bereitgestellt wird. Bei einem sicheren Prothesenaufbau ist es notwendig, dass zur Einbeugung des Prothesenkniegelenkes ein höheres Beugemoment aufgebracht wird als bei einem dynamischen Aufbau. Wird ein sicherer Prothesenaufbau gewählt, ist z.B. während des Stehens eine erhöhte Stabilität in dem Prothesenkniegelenk vorhanden, was für den Prothesenträger angenehm sein kann. Wird ein sicherer Prothesenaufbau gewählt, kann dieser durch eine daran angepasste Erhöhung des Beugemomentes dynamisiert werden, was während des Gehens eine spürbare Erleichterung darstellt.

Das Beugemoment oder Streckmoment wird vorteilhafterweise nur bis zu einem vorbestimmten, ermittelten Kniewinkel aufgebracht. Der Kniewinkel wird durch einen Sensor detektiert, das jeweilige Moment wird vor oder nach Erreichen des festgelegten Kniewinkels reduziert oder abgeschaltet.

Nach dem Einleiten des Beugemomentes kann eine passive Dämpfung erfolgen, um zu verhindern, dass die Unterschenkelkomponente ungedämpft die Einbeugung fortsetzt. Wird für den Antrieb zumindest eine Hydraulikeinheit zwischen dem Oberteil und dem Unterteil, die einen in einem Gehäuse mit einer Extensionskammer und einer Flexionskammer beweglichen Kolben aufweist, der mit dem Oberteil oder dem Unterteil gekoppelt ist, und der eine Druckbereitstellungseinrichtung mit einer Pumpe und einem Druckspeicher zugeordnet ist, über die der Kolben von einer Steuereinrichtung gesteuert mit einem Druck beaufschlagt wird, eingesetzt, ist es ebenfalls möglich, dass die Pumpe im Generatorbetrieb betrieben wird, um die verbrauchte Energie zur Bereitstellung des Momentes wieder aufzufangen und in einem Druckspeicher zu speichern.

Weiterhin ist es möglich, dass die Pumpe im Generatorbetrieb betrieben wird, um Bewegungsenergie in elektrische Energie umzuwandeln, die dann gespeichert und zu einem späteren Zeitpunkt eingesetzt werden kann. Das Betreiben der Pumpe im Generatorbetrieb kann auch als Druckbegrenzung eingesetzt werden, so dass das Hydraulikfluid nicht ohne Druckminderung in den Druckspeicher gelangen kann. Dadurch ist eine geregelte Befüllung mit Druckbegrenzung bei gleichzeitiger Ausnutzung der gesamten Bewegungsenergie beim abwärts Gehen möglich.

Eine Verringerung des Streckmomentes kann vor Erreichen des Streckanschlages eingeleitet werden, um zu verhindern, dass der Unterschenkel ungebremst und mit Unterstützung der Druckbereitstellungseinrichtung in den Streckanschlag fährt.

Beim alternierenden Treppensteigen kann die Druckbereitstellungseinrichtung das Streckmoment dergestalt aufbringen, dass der resultierende Kraftvektor vor der Drehachse des Kniegelenkes gehalten wird. Die Aufbringung eines Streckmomentes beim Treppaufgehen unterstützt den Nutzer. Es ist dabei vorteilhaft, wenn der Kraftvektor kurz vor der Drehachse oder dem Drehpunkt des Kniegelenkes gehalten wird, so dass ein Einbeugen in der Streckphase beim Treppaufgehen nicht stattfinden kann.

Die Druckbereitstellungseinrichtung kann in der Standphase das Kniegelenk aktiv mit einem Moment in Streckung halten, um ein Einbeugen zu verhindern oder zu erschweren und die Standsicherheit für den Nutzer zu erhöhen.

Die Druckbereitstellungseinrichtung kann das Steckmoment in der initialen Schwungphase bis zum Erreichen des maximalen Beugewinkels erhöhen und dieses bis zur Bewegungsumkehr aufrecht halten, wobei das Beugemoment bei abnehmenden Beugewinkel wieder reduziert wird. Die Druckbereitstellungseinrichtung wirkt dabei wie ein Federvorbringer, der einer Beugebewegung entgegenwirkt, bis der maximale Beugewinkel erreicht wird, und die bei der Beugung aufgenommene Energie in der Streckphase wieder abgibt, wobei die Druckbereitstellungseinrichtung das Beugemoment in der Streckbewegung bei zunehmender Streckung reduziert. Eine aktive Unterstützung des Vorbringens in der Schwungphase ist möglich, aber nicht unbedingt vorgesehen.

Neben einem hydraulischen oder pneumatischen Antrieb über eine Pumpe, ggf. mit zumindest einem Druckspeicher, ist auch ein direkter Antrieb über einen Elektromotor oder andere aktuatorische Unterstützung, z.B. ein piezoelektrischer Antrieb, vorgesehen, um ein Moment aufzubringen, mit dem eine Beugung oder Streckung der Gelenkeinrichtung unterstützt werden kann. Die Beaufschlagung mit einem Moment in einer Größe unterhalb einer aktiven Bewegung bewirkt, dass der Nutzer der Kniegelenkeinrichtung die vollständige Kontrolle über die Bewegungseinleitung, die Bewegungsausführung und die Beendigung der Bewegung behält, was zu einer größeren Sicherheit bei der Nutzung führt. Die Energie oder das Moment wird vorteilhafterweise auch nach Beginn der Bewegung weiterhin zugeführt, um die Bewegungsausführung zu erleichtern, dabei kann die Energie in unverminderter oder verminderter Höhe zugeführt werden. Vor dem Ende der Bewegung wird die überschüssige Energie durch Dämpfung umgewandelt, um die Beugung nicht zu weit auszuführen oder das Gelenk bei einer Streckung nicht ungebremst in den Streckanschlag zu fahren. Die Erfindung ist insbesondere vorteilhaft beim langsamen Gehen und bei Prothesenträgern mit kurzen Oberschenkelstümpfen. Die Erfindung vermindert die am Schaft auftretenden Kräfte und sorgt für ein harmonisches Gangbild.

Nachfolgend werden Ausführungsbeispiele der Erfindung anhand der beigefügten Figuren näher erläutert. Es zeigen:
- Figur 1: eine schematische Darstellung einer Gelenkeinrichtung;
- Figur 2: schematische Darstellung einer nicht von der Erfindung umfassten Hydraulikeinheit;
- Figur 3: ein Timingdiagramm zur Gehunterstützung;
- Figur 4: ein Timingdiagramm zur aktiven Vorbringerunterstützung;
- Figur 5: ein Timingdiagramm für das Treppensteigen;
- Figur 5a: eine Variante des Timingdiagramms für das Treppensteigen;
- Figur 6: eine Variante der Schaltungsanordnung;
- Figur 7: eine weitere Variante der Schaltungsanordnung;
- Figur 8: eine Variante der Schaltungsanordnung nach Figur 7;
- Figuren 9 bis 12: Steuerungsabläufe für verschiedene Gehsituationen; sowie
- Figur 13: ein Timingdiagramm zur Beuge- und Streckunterstützung.

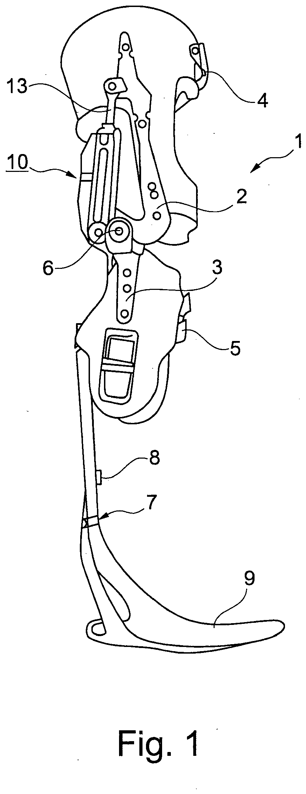

In der Figur 1 ist eine orthetische Gelenkeinrichtung 1 als Teil einer Beinorthese dargestellt. Die Gelenkeinrichtung 1 weist ein Oberteil 2 und ein gelenkig daran angeordnetes Unterteil auf. An dem Oberteil 2 und dem Unterteil 3 sind Befestigungseinrichtungen 4, 5 angeordnet, die als Manschetten oder Hülsen ausgebildet sind. In dem dargestellten Ausführungsbeispiel werden die Manschetten und Hülsen an dem Oberschenkel und dem Unterschenkel des Nutzers der Orthese festgelegt. Das Oberteil 2 ist gegenüber dem Unterteil 3 relativ um eine Schwenkachse 6 verschwenkbar gelagert. An dem Unterteil 3 ist ein Fußteil angeordnet, an dem Sensoren 7, 8 vorgesehen sein können, um die Position des Unterteils 3, die darauf einwirkenden Kräfte oder Momente oder Geschwindigkeiten zu ermitteln. Zwischen dem Oberteil 2 und dem Unterteil 3 ist eine Hydraulikeinheit 10 angeordnet, die nachfolgend näher beschrieben werden wird. In der Hydraulikeinheit 10 ist eine Kolbenstange 13 angeordnet, über die eine Verlagerung des Oberteils 2 relativ zu dem Unterteil 3 die Drehachse 6 bewirkt wird. Ein Fußteil 9 ist am unteren Ende des Unterteils 3 angeschlossen, um einen Fuß aufnehmen zu können. Die Erfindung lässt sich statt einer Orthese auch mit einer Prothese realisieren, bei der die Befestigungseinrichtungen an einem Prothesenstrumpf am Oberteil befestigt sind, während an dem Unterteil der Gelenkeinrichtung weitere prothetische Komponenten angeordnet sind, beispielsweise ein Unterschenkelschaft und ein Prothesenfuß. Entsprechende Anordnungen können für Gelenkeinrichtungen an Hüften oder oberen Extremitäten vorgesehen und ausgebildet sein.

Zur Unterstützung der jeweiligen Bewegung, also der Beuge- und Streckbewegung ist in der Hydraulikeinheit ein Antrieb vorgesehen, über den die jeweilige Bewegung ausgeführt, eingeleitet oder unterstützt wird.

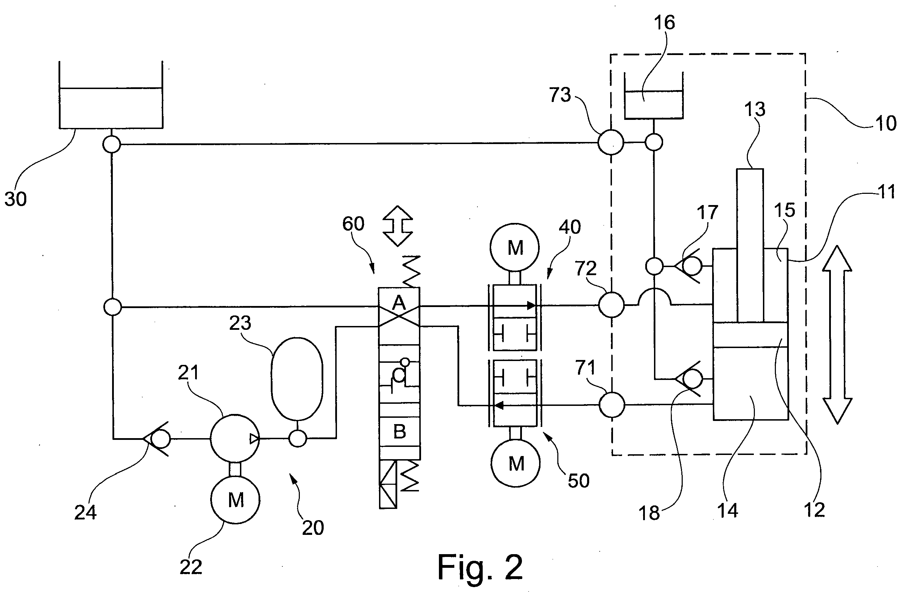

In der Figur 2 ist eine schematische Darstellung der Hydraulikeinheit 10 mit zusätzlichen Komponenten dargestellt. Die Hydraulikeinheit 10 weist ein Gehäuse 11 mit einem beweglich daran gelagerten Kolben 12 auf, der über eine Kolbenstange 13 mit der Gelenkeinrichtung verbunden ist. Das Gehäuse 11 ist mit der jeweils anderen Komponente der Gelenkeinrichtung 1 gekoppelt. In dem Gehäuse 11 wird durch den Kolben 12 eine Extensionskammer 14 von einer Flexionskammer 15 getrennt. Ein Ölspeicher 16 ist über entsprechende Leitungen und Rückschlagventile 17, 18 mit der Extensionskammer 14 und der Flexionskammer 15 verbunden.

Der Hydraulikeinheit 10 zugeordnet ist eine Druckbereitstellungseinrichtung 20, mit der das hydraulische System der Hydraulikeinheit 10 mit Hydraulikfluid versorgt wird. Eine Hydraulikpumpe 21, die von einem Motor 22 angetrieben ist, versorgt die Hydraulikeinheit 10 mit druckbeaufschlagtem Hydraulikfluid. Ebenfalls ist ein Druckspeicher 23 vorgesehen, der ebenfalls druckbeaufschlagtes Hydraulikfluid in das hydraulische System einbringt.

Die Druckbereitstellungseinrichtung 20 ist über eine Umschalteinrichtung 60 in Gestalt eines Schaltventils mit der Hydraulikeinheit 10 verbunden. Die Umschalteinrichtung 60 der dargestellten Ausführungsform ist in drei Stellungen schaltbar, so dass drei unterschiedliche Strömungsverläufe realisiert werden können, die später näher erläutern werden.

Zwischen der Umschalteinrichtung 60 und der Hydraulikeinheit 10 sind verstellbare Ventile 40, 50 angeordnet, über die die Extensionsdämpfung und die Flexionsdämpfung eingestellt werden können. Um die Hydraulikeinheit 10 anzutreiben, ist es sinnvoll, die jeweiligen Dämpfungen möglichst gering zu halten, damit wenige Strömungsverluste auftreten. Die Extensionsdämpfung wird über das Extensionsventil 40 eingestellt, die Flexionsdämpfung über das Flexionsventil 50. Über Motoren ist es möglich, entweder den Fluidstrom variabel zu beschränken oder ganz zu sperren.

Drucksensoren 71, 72, 73 sind vorgesehen, um in Abhängigkeit von den vorhandenen Drücken und den gewünschten Bewegungen oder Dämpfungen die Ventile 40, 50 einzustellen.

Die dargestellte Schaltstellung C ist für eine passive Bewegungsdämpfung vorgesehen, bei der die Druckbereitstellungseinrichtung 20 von der Hydraulikeinrichtung 10 getrennt ist. Eine Extensionsdämpfung und eine Flexionsdämpfung erfolgt über die Einstellung der jeweiligen Ventile 40, 50. ist die Stellung der Umschalteinrichtung so gewählt, dass ein Antrieb durch die Druckbereitstellungseinrichtung 20 nicht möglich ist.
Wird eine Flexionsbewegung durchgeführt, wird der Kolben 12 nach unten gedrückt. Das Hydraulikfluid strömt durch das Flexionsventil 50. Aus der Extensionskammer 14 strömt das Fluid durch das Flexionsventil 50 über den Speicher 16 zurück in die Flexionskammer 15. Aufgrund der durch die Kolbenstange 13 auftretenden Verringerung des rückströmbaren Volumens erhöht sich der Pegel in dem Ölspeicher 16. da keine Unterstützung durch die Pumpe 21 oder den Druckspeicher 23 erfolgt, liegt eine passive Flexion vor.

Eine passive Extension erfolgt bei einer Umkehrung der Bewegung, wenn der Kolben 12 sich nach oben bewegt und aus der Flexionskammer 15 das Hydraulikfluid durch das Extensionsventil 40 hindurch in dem Strömungskreislauf durch die Umschalteinrichtung 60, wobei Hydraulikfluid aus dem Speicher 16 der Hydraulikeinrichtung 10 zusätzlich in die Extensionskammer 14 strömt.

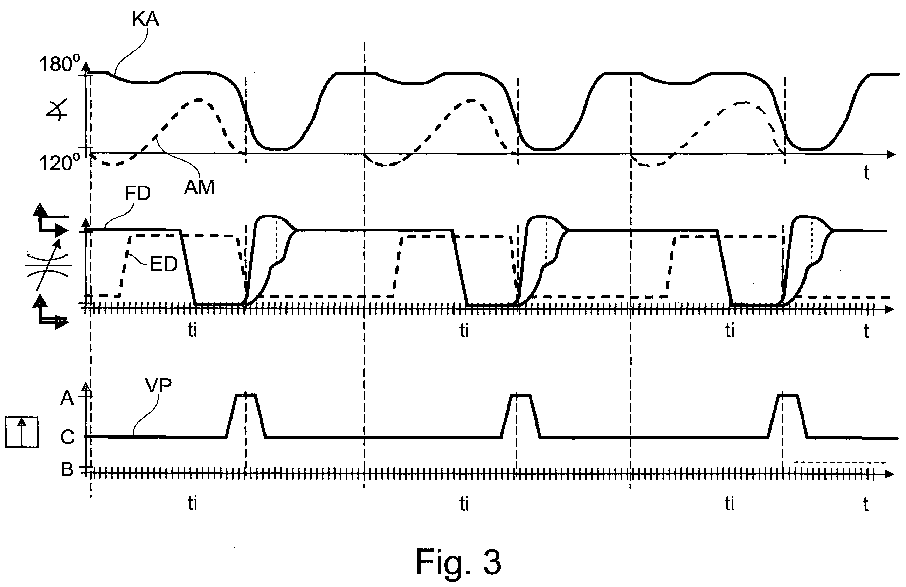

In der Figur 3 sind drei Diagramme über die Zeit dargestellt. Das obere Diagramm zeigt den Kniewinkel KA und das Knöchelmoment AM über die Zeit während eines normalen Gehens in der Ebene. Das mittlere Diagramm zeigt die Flexionsdämpfung FD und die Extensionsdämpfung ED über die Zeit während des normalen Gehens, das untere Diagramm zeigt die Ventilstellung VP zu dem jeweiligen Zeitpunkt. Die Diagramme sind für die Ausgestaltung der Gelenkeinrichtung als ein Prothesenkniegelenk dargestellt. Die Diagramme beginnen zu dem Zeitpunkt, in dem die Ferse aufsetzt, also zu dem Zeitpunkt des sogenannten "heel-strike". Der Kniewinkel KA beträgt 180°, das Knöchelmoment AM wirkt in Plantarrichtung und verläuft somit unterhalb der waagerechten Linie. Die Flexionsdämpfung FD ist hoch, die Extensionsdämpfung ED niedrig, bis zu dem Zeitpunkt, zu dem das Knöchelmoment AM positiv wird. Dann wird die Extensionsdämpfung ungefähr auf das Niveau der Flexionsdämpfung FD angehoben und bleibt dort bis zum erneuten Schwingen nach vorn. Die Flexionsdämpfung FD bleibt hoch, bis zum Zeitpunkt des Ablösens des Vorderfußes, dem sogenannten "toe off". Dann wird die Flexionsdämpfung FD abgesenkt, um ein Schwingen des Unterschenkels nach hinten zu ermöglichen. Bis kurz vor dem Flexionsmaximum bleibt die Flexionsdämpfung niedrig und wird kurz vor Erreichen des Flexionsmaximums, also des niedrigsten Kniewinkels KA, wieder erhöht, um einerseits die Flexionsbewegung zu bremsen und andererseits möglichst schnell eine maximale Sicherheit gegen ein ungewolltes Einknicken zu erhalten. Gleichzeitig wird die Extensionsdämpfung ED verringert, um eine möglichst zügige Vorwärtsbewegung des Unterschenkels und des Prothesenfußes zu ermöglichen. Während der Standphase ist die Ventilstellung VP in der Stellung C, in der eine passive Flexion stattfindet. Kurz vor Beginn des Flexionsmaximums wird die Umschalteinrichtung 60 in die Stellung A bewegt, um eine Flexionsunterstützung bereitzustellen. Die Beugeunterstützung wird ausgestaltet, sobald ein gewisser Kniewinkel erreicht wird. Eine gesteuerte Extensionsdämpfung ED sorgt für eine Vermeidung des so genannten "heel rising". Die aktive Beugeunterstützung wird anhand des Verlaufs des Knöchelmomentes AM erkannt.

Um eine präzise Steuerung der Hydraulikeinheit 10 mit den Ventilen 40, 50 und der Umschalteinrichtung 60 zu gewährleisten, sind Sensoren vorgesehen, die die einzelnen Komponenten überwachen. Neben einem Kniewinkelsensor und einem Knöchelmomentsensor können auch Axialkraftsensoren vorgesehen sein. Die Steuerung kann beispielsweise über die Betrachtung der Gelenkwinkelgeschwindigkeit erfolgen. Bei einem Wendepunkt der Kniewinkelgeschwindigkeit wird eine aktive Flexionsunterstützung mit der Ventilstellung A für einen gewissen Zeitraum aktiviert. Das Flexionsventil 50 wird auf einen niedrigen Wert eingestellt und bei Überschreiten eines definierten Gelenkwinkels wird die Unterstützung abgeschaltet und die Umschalteinrichtung in die Position C verlagert.

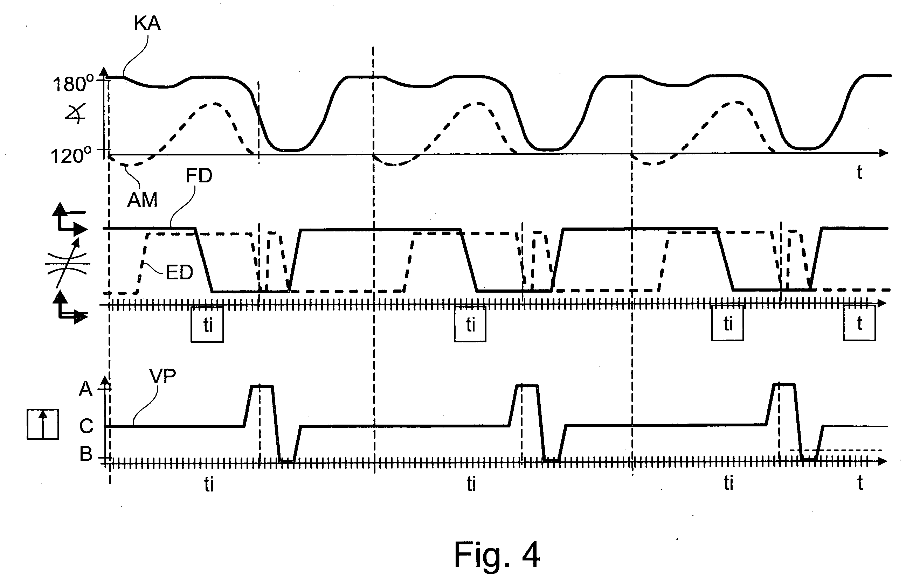

Eine Variante der Steuerung ist in der Figur 4 dargestellt, in der die Unterstützung mit einer aktiven Vorbringerunterstützung dargestellt ist. Bis zum Abheben des Fußes, was am Knöchelmoment AM erkannt werden kann, verläuft die Steuerung wie gemäß Figur 3 dargestellt. Statt nach dem Abheben wieder in die Stellung C für die Umschalteinrichtung 60 zu verfahren, wird die Ventilposition B eingenommen und die Extensionsdämpfung ED erhöht. In der Ventilstellung B der Umschalteinrichtung 60 wird die Extension unterstützt, also die Kniewinkelgeschwindigkeit verringert und die Drehrichtung umgekehrt. Für ein sanftes Zuschalten der Extensionsunterstützung kann das Extensionsventil 40 zu passenden Zeitpunkten geschlossen und wieder geöffnet werden. Ein sanftes Abschalten der Extensionsunterstützung wird durch das Schließen des Flexionsventils 50 erreicht.

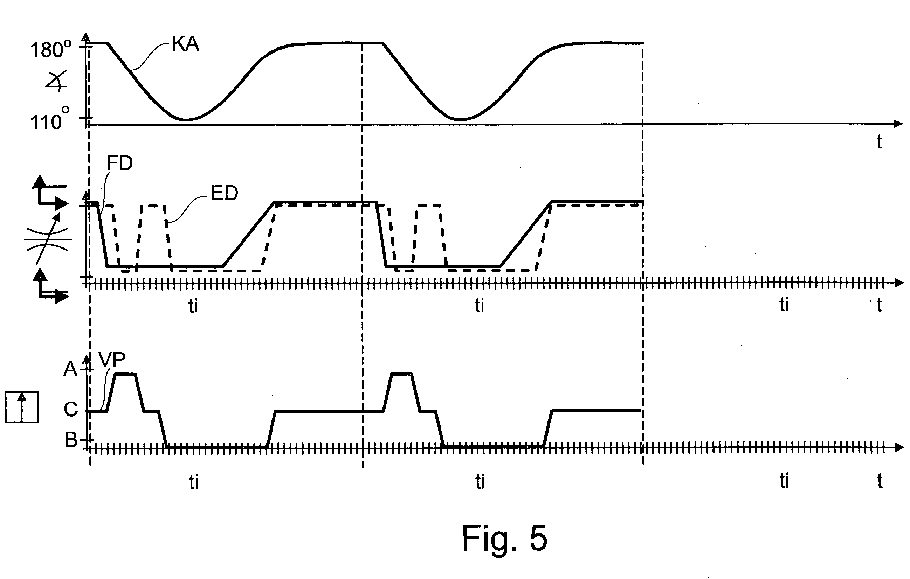

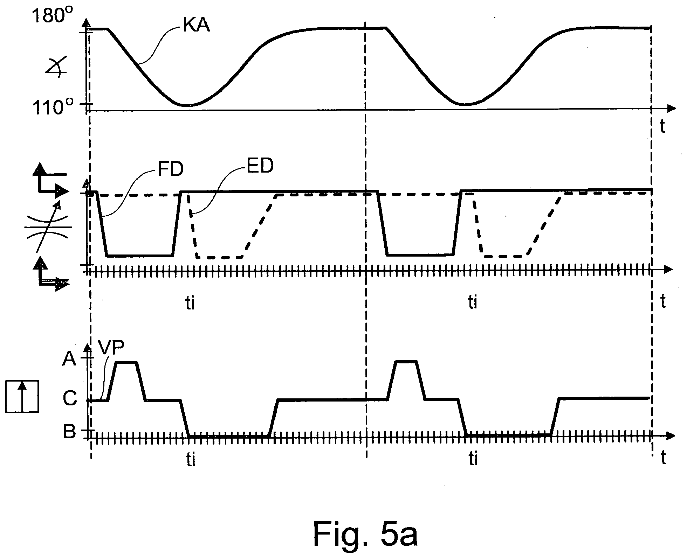

In der Figur 5 sind die Diagramme für das Treppensteigen aufgezeichnet. Ein Knöchelmoment liegt nicht vor. Nach dem Anheben des Fußes verringert sich der Kniewinkel KA. Um dies zu erleichtern, wird die Extensionsdämpfung ED verringert, die Umschalteinrichtung 60 verfährt in die Position A, um eine Flexionsunterstützung zu erwirken. Die Flexionsdämpfung FD bleibt weiterhin niedrig. Die Extensionsdämpfung ED wird für den Zeitraum der aktiven Flexion ebenfalls verringert. Anschließend wird die Umschalteinrichtung 60 in die Position C verfahren, um eine passive Flexion mit einer Abbremsung der Flexionsbewegung zu erreichen, bis der Kniewinkel KA minimal ist. Abschließend wird die Umschalteinrichtung 60 in die Ventilposition B verfahren, durch eine aktive Extension erreicht wird, so dass die Unterstützung durch die Druckbereitstellungseinrichtung 20 wirksam werden kann. Für die Dauer der Umschaltung der Umschalteinrichtung 60 wird die Extensionsdämpfung ED wieder erhöht, in der Ventilstellung B, bei der eine aktive Streckung unterstützt wird, wird die Extensionsdämpfung ED reduziert, um die volle Wirksamkeit der Unterstützung zu gewährleisten. Die Flexionsdämpfung FD wird während des Anhebens des Patienten in Abhängigkeit von dem Kniewinkel KA langsam erhöht, wodurch das unterstützende Moment kleiner wird und ein harter Anschlag in den Extensionsanschlag vermieden wird. Zusätzlich wird bei einem fast gestreckten Kniegelenk die Extensionsdämpfung ED erhöht, um einen harten Anschlag ebenfalls zu dämpfen.

In der Figur 5a ist eine Variante des Timingdiagramms gemäß Figur 5 dargestellt. Die Steuereinrichtung erkennt die Situation, dass eine Stufe erklommen werden soll. Ausgehend von der Stellung C wird die Umschalteinrichtung 60 ab einem definierten Kniewinkel in die Stellung A verfahren, um eine aktive Beugeunterstützung zu erreichen. Der Patient erhält damit eine Unterstützung, um das versorgte Bein für die Stufe einzubeugen, damit der Prothesenfuß nach hinten schwingen kann, um nicht an der Unterkante einer Treppenstufe hängen zu bleiben. Um die Wirksamkeit der aktiven Beugeunterstützung zu gewährleisten, wird die Flexionsdämpfung FD reduziert. Die Extensionsdämpfung bleibt auf dem hohen Ausgangsniveau.

Ab einem definierten Zielwinkel des Kniewinkels KA wird die Flexionsunterstützung abgeschaltet und die Umschalteinrichtung 60 kehrt in die Stellung C zurück. Die Flexionsdämpfung FD und die Extensionsdämpfung ED bleiben unverändert. Kurz vor Erreichen des maximalen Kniewinkels wird die Flexionsdämpfung erhöht, um den maximalen Kniewinkel KA zu begrenzen. Nach dem Erreichen des maximalen Flexionswinkels und dem Aufsetzen des Fußes wird eine Extensionsunterstützung aktiviert und die Umschalteinrichtung 60 in die Ventilstellung B verfahren. Gleichzeitig wird die Extensionsdämpfung ED reduziert, um eine volle Unterstützung der Anhebebewegung zu gewährleisten. Die Flexionsdämpfung wird auf hohem Niveau belassen, beispielsweise um ein Rückfallen des Patienten zu verhindern. Beim Anheben des Patienten wird in Abhängigkeit von dem Kniwinkel KA die Extensionsdämpfung ED langsam erhöht, um das unterstützende Moment durch die Pumpe 21 oder den Druckspeicher 23 zu verkleinern und einen harten Anstoß in den Extensionsanschlag zu vermeiden. Nach Erreichen des maximalen Streckwinkels wird die Unterstützung abgeschaltet und die Ventilstellung B eingeschaltet.

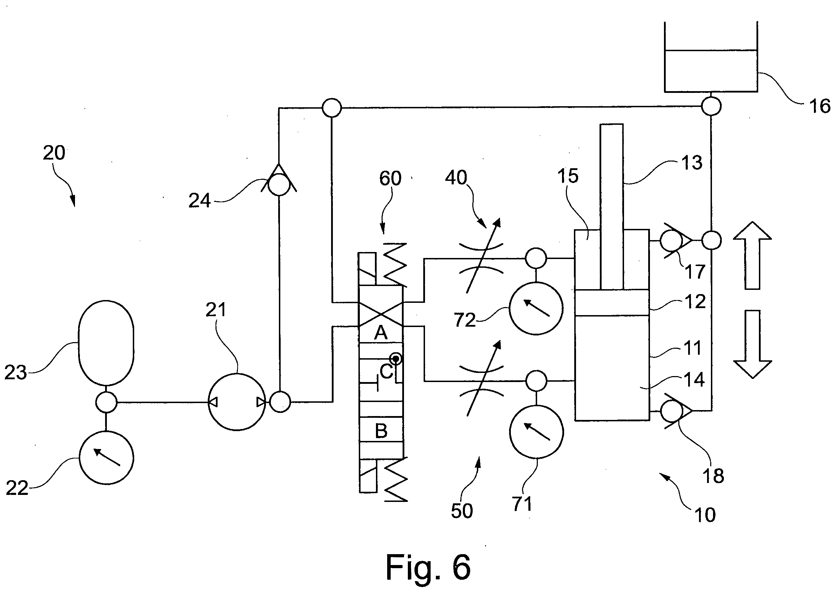

In der Figur 6 ist eine Variante der aktiven Flexionsunterstützung dargestellt. Die Hydraulikeinheit 10 ebenso wie die Umschalteinrichtung 60 entsprechen im Wesentlichen denen der Figur 2. Die Druckbereitstellungseinrichtung 20 ist jedoch auf eine andere Art und Weise mit den übrigen Komponenten gekoppelt. Die Pumpe 21 als eine Pumpe ausgebildet, die in beide Richtungen fördern kann, so dass auch der Druckspeicher 23 aufladen kann. Der Druckspeicher 23 ist mit einer Stichleitung mit der Pumpe 21 verbunden. Dadurch ist es möglich, dass eine separate Druckbeaufschlagung des Druckspeichers 23 vorgenommen werden kann. Wird der Druckspeicher 23 entladen, also wird Hydraulikfluid aus dem Druckspeicher 23 in Richtung auf die Hydraulikeinheit 10 gefördert, kann die Pumpe 21 mit dem Motor den Druck zusätzlich erhöhen. Dabei ist es möglich, zwei unterschiedliche Drücke zu realisieren, da eine Flexionsunterstützung in der Regel weniger Druck als eine Extensionsunterstützung benötigt. Darüber hinaus ist es möglich, die Pumpe 21 als Generator einzusetzen. Ist der Druck im Druckspeicher 23 wesentlich höher als er innerhalb des Hydraulikzylinders der Hydraulikeinheit 10 benötigt wird, kann die Pumpe 21 in einem Generatorbetrieb betrieben werden, um eine Druckminderung zu bewirken und gleichzeitig einen Teil der hydraulischen Energie in elektrische Energie umzuwandeln. Die hydraulische Energie würde sonst an den jeweiligen Ventilen 40, 50 verloren gehen, da der Druck immer sanft zugeschaltet werden soll. Durch eine Steuerung der Generatorenergieabgabe kann mit variablen Druckunterschieden gearbeitet werden, wodurch sich die Verlustleistung und die Umwandlung der Druckenergie in Wärme minimieren lässt.

Darüber hinaus ist es mit der Schaltung gemäß Figur 6 möglich, dass der Druck im Speicher 23 wesentlich erhöht werden kann, ohne dass dies zu erhöhten Verlusten an den Ventilen 40, 50 führen würde. Der Speicher 23 kann somit mehr Energie aus den Bewegungen der Gelenkeinrichtung speichern. Bei der Durchleitung des Hydraulikfluids aus dem Druckspeicher 23 zu der Hydraulikeinheit kann die Pumpe 21 im Generatorbetrieb betrieben werden, um eine Anpassung des gewünschten Hydraulikdruckes zu ermöglichen. Die Reduzierung des Druckes erfolgt durch Umwandlung in elektrische Energie, die gespeichert und zum Betreiben der Pumpe 21 verwendet werden kann.
Sofern keine aktive Unterstützung für die Extensionsbewegung notwendig ist, kann diese Stellung der Steuereinrichtung 60 auch dazu benutzt werden, den Druckspeicher 23 mit dem Hydraulikfluid zu beaufschlagen. Dazu wird der Druck in dem Druckspeicher 23 über den Drucksensor 22 gemessen, gleichzeitig wird der Druck am Hydraulikzylinder 11 am Ausgang der Flexionskammer 15 gemessen. Der Drucksensor 72 zeigt an, ob ein Druckunterschied zwischen dem Druckspeicher 23 und der Flexionskammer 15 vorhanden ist. In Abhängigkeit des Druckunterschiedes wird das Flexionsventil 40 eingestellt. Ist kein Druck in dem Druckspeicher 23 vorhanden, würde ein Prothesennutzer bei einer vollständigen Öffnung des Flexionsventils 40 keinen Widerstand erfahren und stürzen. Das Flexionsventil 40 wird daher in Abhängigkeit der Drücke so geschaltet, dass der Nutzer der Gelenkeinrichtung immer denselben Widerstand beim Treppabgehen spürt. Ähnliches gilt für das Abwärtsgehen auf einer schrägen Ebene. Der Druckspeicher 23 lädt sich bei jedem Schritt bergab weiter auf und wird dadurch mit Druck beaufschlagt.

Als weitere Option kann ein weiterer Druckspeicher vorgesehen sein, der für sehr hohe Drücke ausgelegt ist, höher als diejenigen Drücke, die der Druckspeicher 23 für einen normalen Betrieb der Gelenkeinrichtung benötigt. Der zusätzliche Speicher kann durch das Bergabgehen oder Treppabgehen sehr hoch aufgeladen werden, so dass sich das Energiespeicherpotenzial erhöht. Es besteht die Möglichkeit, dass damit die Pumpe 21 als ein pneumatischer Motor betrieben wird, so dass im Generatorbetrieb elektrische Energie erzeugt werden kann. Der zusätzliche Speicher könnte auch gesteuert in den Druckspeicher 23 entladen werden, um Druckreserven kreieren zu können.

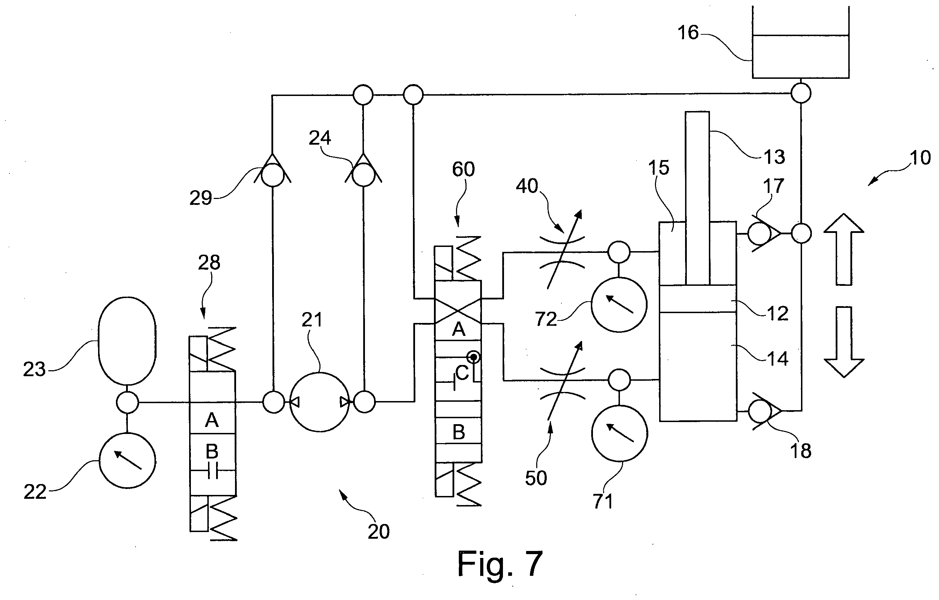

In der Figur 7 ist eine Variante der Figur 6 dargestellt, die vorsieht, dass zwischen der Pumpe 21 bzw. dem Generator und dem Druckspeicher 23 ein Schaltventil 28 angeordnet ist, mit dem es möglich ist, den Druckspeicher 23 von den übrigen Komponenten abzukoppeln. Dadurch ist es möglich, die Pumpe 21 unabhängig von dem Druckspeicher 23 zu betreiben. Hierzu ist ein weiteres Rückschlagventil 29 vorgesehen, das parallel zu dem bereits vorhandenen Rückschlagventil 24 angeordnet ist. Beide Rückschlagventile 24, 29 verhindern, dass aus der Pumpe 21 Hydraulikfluid in den Speicher 16 gefördert wird.

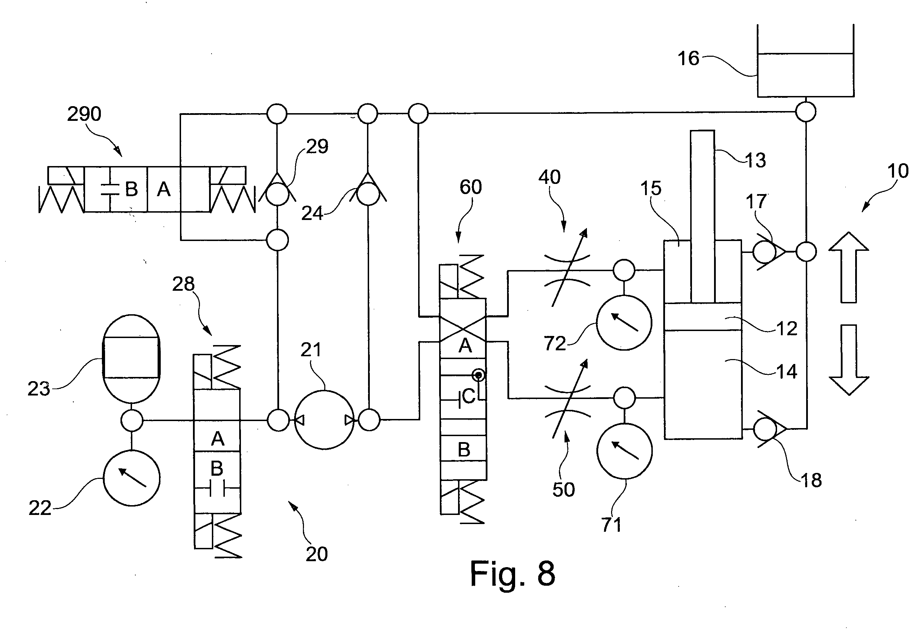

In der Figur 8 ist eine Variante der Ausführungsform gemäß Figur 7 dargestellt. Die Ausführungsform gemäß Figur 8 sieht vor, dass eine Schaltventileinrichtung 290 parallel zu dem Rückschlagventil 29 in der Hydraulikleitung angeordnet ist. Diese Ventileinrichtung 290 ermöglicht es, das Rückschlagventil 29 zu umgehen, wenn sie sich in der Schaltstellung A befindet. Diese Schaltstellung ist dargestellt. In der Schaltstellung B der Ventileinrichtung 290 ist der Bypass gesperrt, so dass Hydraulikfluid nicht durch das Rückschlagventil 29 durchströmen kann, wenn ein entsprechendes Druckgefälle an dem Ventil 29 anliegt. Diese zusätzliche Ventileinrichtung 290 dient dazu, dass bei einem gefüllten Druckspeicher 23 ein durch eine Kniebeugung erzwungener Ölstrom über die Pumpe 21 in einem Generatormodus zur Stromerzeugung genutzt werden kann. Dazu sind die Ventileinrichtungen 290, 28, 60 in die entsprechenden Stellungen zu verfahren, so dass die Pumpe 21 im Generatorbetrieb bei der Flexion durch das Hydraulikfluid angetrieben werden kann. Durch den Generatorbetrieb wird eine ggf. zusätzliche Flexionsdämpfung durch die Umwandlung der mechanischen und hydraulischen Energie in elektrische Energie erreicht.

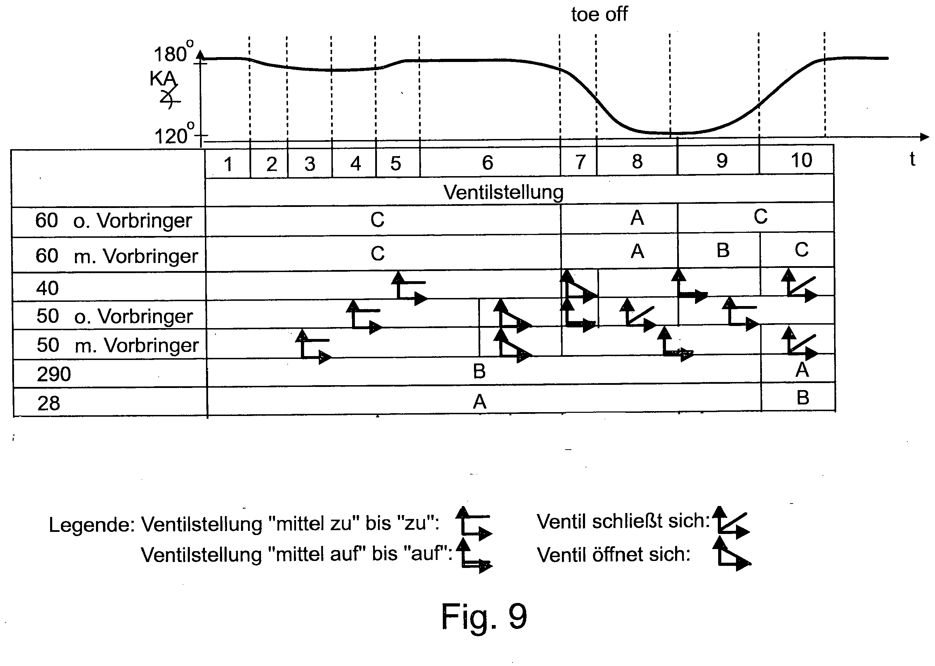

In der Figur 9 ist tabellarisch die Stellung der einzelnen Ventile oder Steuereinrichtungen und deren Veränderungen über den Verlauf eines Schrittes gezeigt. Oberhalb der Tabelle ist ein Schrittzyklus für das Gehen in der Ebene dargestellt. Der übliche Kniewinkel KA verläuft zwischen ungefähr 180° in der maximal gestreckten Stellung und ca. 120° in der maximal flektierten Stellung. Ein Gangzyklus lässt sich in mehrere Phasen unterteilten, die wichtigste Unterteilung erfolgt zwischen der Standphase und der Schwungphase. Dargestellt ist der Kniewinkelverlauf beginnend mit dem Fersenstoß, also dem Aufsetzen eines Prothesenfußgelenkes, an dem sich eine Standphasenflexion anschließt. Nach dem ersten Bodenkontakt, in dem sich das Kniegelenk in einer stabilen Stellung befindet, wird durch die Belastung durch das Körpergewicht das Kniegelenk eingebeugt. Die unmittelbar auf die Belastung folgende Flexion dämpft den Impuls durch den Bodenkontakt. Der initiale Bodenkontakt und die Belastungsantwort erfolgen in den Zeiträumen 1 bis 4. Anschließend wird eine Extension des Kniegelenks eingeleitet, um eine verbesserte Stabilität zu erreichen. Anschließend wird die Extension vervollständigt. Dies sind die mittleren und terminalen Standphasen in dem sechsten Zeitraum. Anschließend erfolgt eine passive Flexion des Kniegelenkes in der sogenannten Vorschwungphase 7. Am Ende der Vorschwungphase 7 erfolgt der sogenannte "toe-off", also das Abheben des Fußes von dem Boden, damit das Bein vorschwingen kann. In der initialen Schwungphase wird dann anschließend die maximale Flexion erreicht, in der mittleren Schwungphase 9 wird das Vorschwingen des Beines erreicht und in der terminalen Schwungphase wird die Extension des Kniegelenkes vorangetrieben, um den maximalen Kniewinkel zu erreichen und die Vorbereitung auf die Standphase durchzuführen.

In der unter diesem Bewegungsablauf aufgeführten Tabelle sind die einzelnen Elemente der Hydraulikschaltung der Gelenkeinrichtung für die Situation mit einer aktiven Extensionsunterstützung und ohne eine aktive Extensionsunterstützung dargestellt. Bis zur terminalen Standphase ist die Umschalteinrichtung 60 in der mittleren Stellung C angeordnet, für die terminale Standphase bis zur Maximalflexion wird in die Ventilstellung A gebracht, um eine Unterstützung bei der Beugeeinleitung zu ermöglichen. Nach Erreichen der Maximalflexion wird ohne die Vorbringerfunktion, also eine Extensionsunterstützung, eine Unterstützung durch einen Druckspeicher 23 oder die Pumpe 21 wieder ausgeschaltet. Bei einer Extensionsunterstützung wird nach Erreichen der Maximalflexion in die Ventilstellung B geschaltet, um eine Extensionsunterstützung zu erreichen.

Das Extensionsventil 40 bleibt dabei während der Standphase überwiegend geschlossen. Erst zu Beginn der terminalen Standphase öffnet sich das Extensionsventil, bleibt während der gesamten Flexionsphase im Wesentlichen geöffnet und erst während der terminalen Schwungphase wird das Extensionsventil wieder geschlossen, um einen harten Extensionsanschlag zu vermeiden. Die hohe Extensionsdämpfung während der Standphase verhindert einen harten Anschlag in die Extension während der Standphasenextension.

Das Flexionsventil 50 ist zunächst überwiegend geschlossen, um eine Standphasenbeugung zu dämpfen. Nach der Standphasenextension wird die Flexionsdämpfung verringert, um eine Beugung zu ermöglichen. Vor der Beugeeinleitung wird die Flexionsdämpfung maximal verringert, da hier das Kniegelenk durch die Bodenreaktionskräfte im Anschlag gehalten wird. Ohne eine Extensionsunterstützung wird die Flexionsdämpfung in der Schwungphase erhöht, um ein Überschwingen des Prothesenfußes und das sogenannte "heel rising" zu vermeiden. Die Flexionsdämpfung bleibt auf einem hohen Niveau, um ein Stolpern abfangen zu können. Das Flexionsventil mit Vorbringerfunktion sieht keine Erhöhung der Dämpfung während der Flexionsphase vor, da hier kein "heel rising" vermieden werden muss, da dies durch die Aktivierung der Extensionsunterstützung erfolgt. Die Dämpfung in Flexionsrichtung bleibt länger niedrig im Vergleich zu der Dämpfung ohne eine Vorbringerfunktion, um eine Extensionsunterstützung zu ermöglichen. Anschließend wird die Flexionsdämpfung wieder erhöht, um ein Stolpern abfangen zu können.

Für die Variante gemäß Figur 8 mit den Ventilen 290 und 28 ist vorgesehen, dass das Ventil 290 geschlossen bleibt, um keine Verluste bei der Unterstützung oder Aufladung eines Speichers zu erfahren. Am Ende der terminalen Schwungphase kann das Ventil geöffnet werden, um den Ölstrom durch die Pumpe 21 im Generatorbetrieb zu leiten und damit die mechanische Energie in elektrische Energie umzuwandeln. Das Ventil 28 wird erst während der terminalen Schwungphase geschlossen, um bei einem gefüllten Speicher 23 ein erzwungener Ölstrom über den Generatorbetrieb der Pumpe 21 zur Stromerzeugung zu nutzen.

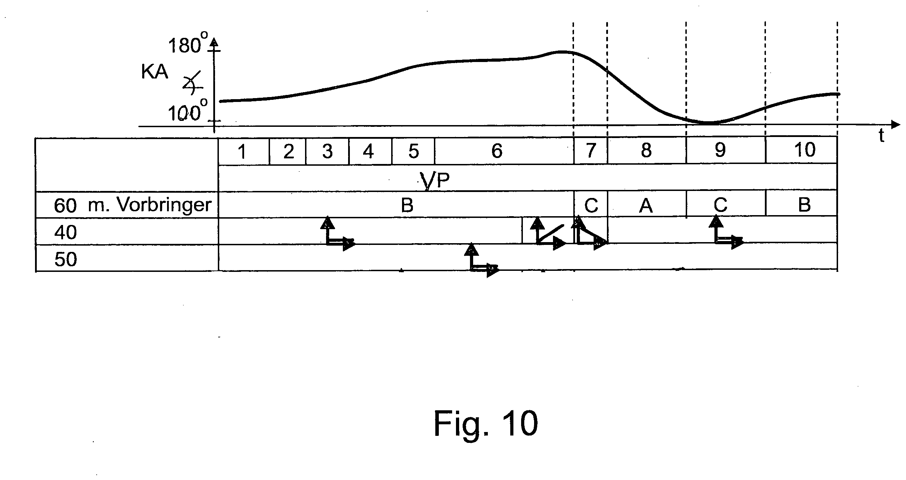

Figur 10 zeigt die Schaltungsanordnung beim Steilaufwärtsgehen. Sinnvollerweise wird hier mit einer Extensionsunterstützung gearbeitet. Bis zur terminalen Standphase ist das Ventil 60 in der Stellung B, um eine Extensionsunterstützung zu gewährleisten, damit der Patient eine Treppenstufe leichter erklimmen kann oder auf einer schrägen Rampe leichter hinaufgehen kann. Eine Flexionsunterstützung wird bei der Beugeeinleitung nach dem toe-off vorgesehen, eine Extensionsunterstützung in der terminalen Schwungphase. Das Extensionsventil 40 bleibt zunächst im Wesentlichen geöffnet, um eine niedrige Dämpfung in Extensionsrichtung zu gewährleisten, damit die Extensionsunterstützung maximal wirksam ist. Vor Erreichen des Streckanschlages ist ein Schließen des Extensionsventils 40 und damit ein Erhöhen der Extensionsdämpfung notwendig, um einen harten Anschlag in den Extensionsanschlag zu vermeiden. An den Umschaltzeitpunkten des Umschaltventils 60 kann ein kurzzeitiges Schließen des Extensionsventils sinnvoll sein, um harte Stöße zu vermeiden, alternativ kann das Flexionsventil 50 an diesen Zeitpunkten kurz geschlossen werden. Ansonsten ist das Flexionsventil 50 über den gesamten Bewegungszeitraum im Wesentlichen geöffnet, um eine minimale Dämpfung und eine maximale Unterstützung zu ermöglichen.

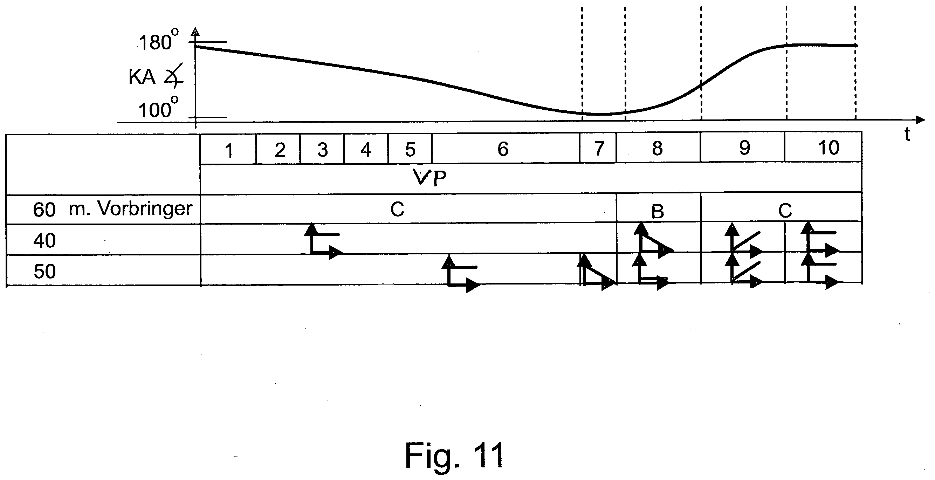

In der Figur 11 ist ein Diagramm für das Steilabwärtsgehen mit Vorbringerfunktion dargestellt. Über die gesamte Standphase bleibt das Umschaltventil in der Stellung C, es findet keine Extensionsunterstützung oder Flexionsunterstützung statt. Erst in der initialen Schwungphase wird die Stellung B eingenommen, um eine Flexionsunterstützung zu erreichen. Das Extensionsventil ist in der Standphase geschlossen oder weist eine hohe Dämpfung auf, um eine hohe Extensionsdämpfung zu gewährleisten, falls doch kein steiler Abstieg mehr vorhanden ist. Nach Erreichen der maximalen Flexion wird die Extensionsdämpfung verringert, um die Extension zu ermöglichen, das Ventil 40 wird somit geöffnet. Am Ende der Schwungphase wird die Extension wieder erhöht, um ein hartes Anschlagen an den Extensionsanschlag zu vermeiden.

Das Flexionsventil 50 weist während der Standphase einen erhöhten Flexionswiderstand auf, um bei der Abwärtsbewegung die Beugung zu dämpfen. Anschließend wird das Flexionsventil 50 langsam geöffnet, um eine Flexionsbewegung zu ermöglichen. In der Schwungphase bleibt die Dämpfung niedrig, um eine Extensionsunterstützung zu ermöglichen, falls dies gewünscht ist, alternativ kann die Flexionsdämpfung auch erhöht werden, wie es im Bereich der mittleren Schwungphase vorgesehen ist. Im Bereich der terminalen Schwungphase ist wieder eine maximale Flexionsdämpfung vorgesehen.

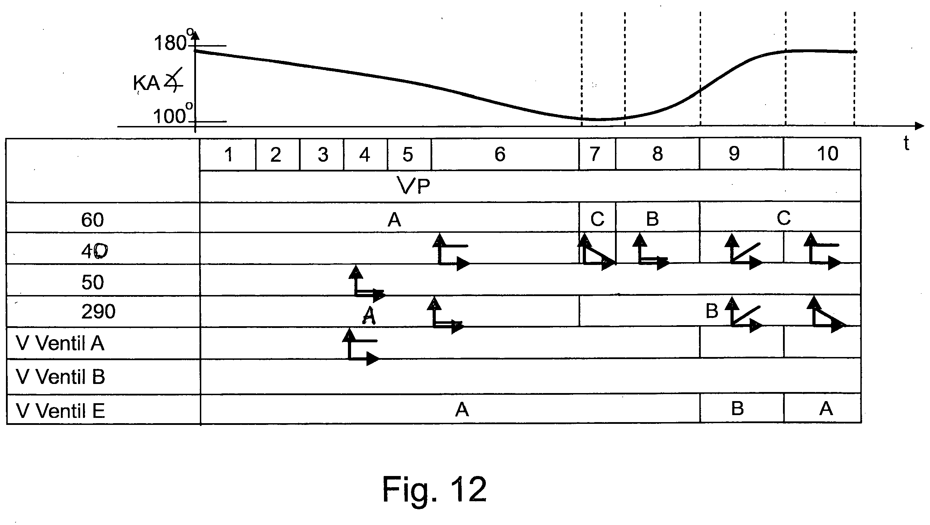

In der Figur 12 ist eine Schaltungsanordnung und ein Schaltverlauf gezeigt, der beim Steilabwärtsgehen gemäß Figur 11 erfolgen kann, wenn der Druckspeicher 23 aufgeladen werden soll bzw. wenn elektrische Energie im Generatorbetrieb der Pumpe 21 generiert werden soll. Das Umschaltventil 60 wird während der Standphase in die Stellung A verfahren, damit Energie zum Speicher 23 geführt werden kann. Dies soll sinnvollerweise nur dann stattfinden, wenn der Speicherdruck dadurch den Kolbendruck nicht übersteigt. Wenn dies der Fall sein sollte, wäre das Schaltschema gemäß Figur 11 anzuwenden.

Abweichend zu der Figur 11 wird in der Steuerung gemäß Figur 12 das Extensionsventil bereits in der terminalen Standphase geöffnet und die Extensionsdämpfung verringert. Über die gesamte initiale Schwungphase bleibt das Extensionsventil geöffnet und wird erst in der mittleren Schwungphase wieder geschlossen, um die Extensionsdämpfung zu erhöhen, damit am Ende der Schwungphase ein harter Anschlag vermieden wird. Das Flexionsventil bleibt über den gesamten Bewegungsablauf geöffnet, um die Energie in den Speicher 23 zu leiten. Die Stellung des Ventils sollte dem Druckunterschied zwischen dem Speicher 23 und dem Kolben angepasst werden. Ist der Speicher 23 leer, kann das Flexionsventil 50 so eingestellt werden, dass eine höhere Flexionsdämpfung vorhanden ist. In der Schwungphase ist die Flexionsdämpfung ebenfalls niedrig zu halten, um eine Extensionsunterstützung zu ermöglichen, falls dies gewünscht ist. Das Schaltventil 290 wird während der Standphase geöffnet, damit der Ölstrom durch den Generator zur Stromgewinnung genutzt werden kann. Das Ventil 28 bleibt geschlossen. In der Extensionsphase wird das Ventil 290 geschlossen, um eine Extensionsunterstützung durch den Druckspeicher 23 zu ermöglichen.

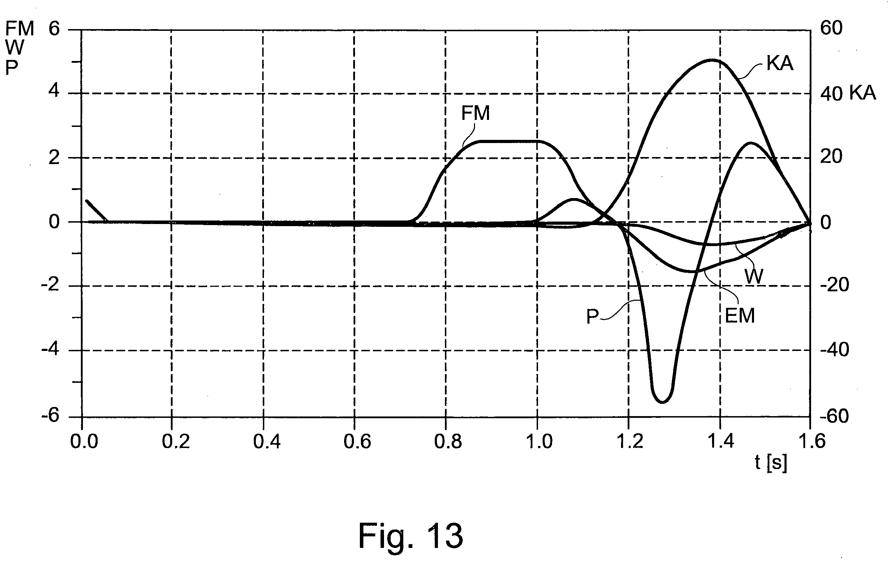

In der Figur 13 ist ein Timing-Diagram für einen Gangzyklus dargestellt, in dem ein Kniewinkel KA über die Zeit t dargestellt ist. Der Kniewinkel KA ist zu Beginn des Gangzyklus', also bei dem Fersenauftritt oder dem sogenannten "heel strike" aufgrund der anfänglichen Standphasenflexion größer als 0°, bleibt über den anschließenden Standphasenabschnitt konstant bei 0°, bis er sich nach ca. 1,1 s erhöht. Der Kniewinkel KA vergrößert sich nach dem Ende der Standphase, nach dem sogenannten "toe-off", weiter bis zu einer maximalen Einbeugung von ca. 55 ° bei t = 1,35 s. Nach Erreichen der maximalen Kniewinkels KA erfolgt eine Bewegungsumkehr, die Unterschenkelkomponente wird nach vorn bewegt, so dass sich der Kniewinkel KA aufgrund der Extensionsbewegung wieder verringert und am Ende der Schwungphase in der maximalen oder angenähert maximalen Streckung bei einem Kniewinkel KA von 0 ° endet. Abweichend von der Darstellung in den anderen Figuren ist der Kniewinkel KA in der Figur 13 in der gestreckten Stellung mit 0° bezeichnet.

Der Figur 13 ist weiter zu entnehmen, dass erfindungsgemäß ein unterstützendes Beugemoment FM unmittelbar vor dem Einbeugen aufgebracht wird. Anhand des Kniewinkelverlaufes ist zu erkennen, dass das Beugemoment FM nicht ausreicht, um eine Kniebeugung zu bewirken, vielmehr ist das Niveau des Beugemomentes FM so gewählt, dass nur eine Unterstützung für eine Einbeugung durch den Nutzer bereitgestellt wird und nicht eigenständig eine Einbeugung bewirkt wird. Dies lässt sich daraus erkennen, dass der Kniewinkel KA über den gesamten Zeitraum des maximalen Beugemomentes FM unverändert bleibt. Bereits vor Beginn der Einbeugung, also einer Veränderung des Kniewinkels in Richtung einer Kniebeugung, wird das Niveau des Beugemomentes FM verringert, um zu vermeiden, dass eine zu leichte Einbeugung des Kniegelenks erreicht wird, was eine mangelnde Stabilität des Kniegelenkes zur Folge haben würde. Die nur zeitweise Aufbringung des Beugemomentes hat den Vorteil, dass weniger Energie bereitgestellt werden muss. Ebenfalls bleibt das Kniegelenk während der Standphase überwiegend ohne Beugeunterstützung, so dass eine hohe Sicherheit gegen ungewolltes Einbeugen gewährleistet ist. Durch die Unterstützung, die als Verringerung des Auslösemomentes zur Einbeugung verstanden werden kann, kann ein stabiler Aufbau gewählt werden, so dass ein erhöhtes Maß an Standsicherheit bereitgestellt werden kann, ohne dass dies aufgrund des sicheren Aufbaus, also der Zuordnung der einzelnen Komponenten zueinander, zu einen erhöhten Belastung für den Patienten führen würde.

Nach dem "toe-off", also bei ca. t = 1,2 s, ist ein unterstützendes Beugemoment nicht mehr vorhanden, vielmehr wird ein Extensionsmoment EM aufgebracht, das als Flexionsdämpfung wirkt und ein übermäßiges Einbeugen des Kniegelenkes verhindert. Statt eines aktiven Aufbringens eines Extensionsmomentes EM kann auch eine rein passive Dämpfung erfolgen, ebenfalls ist es möglich, dass zur Dämpfung Energie aus dem System durch Umwandlung abgeführt wird, so dass die zur Beaufschlagung mit einem Beugemoment benötigte Energie zumindest teilweise rückgeführt werden kann. Wird ein Streckmoment EM aufgebracht, endet die Phase, in der ein Streckmoment EM aufgebracht wird, vor der vollständigen Streckung des Kniegelenkes.

Es ist möglich und vorgesehen, dass die Energie auch über die Auslösung der Bewegung hinaus aufgebracht bleibt, entweder auf dem gleichen oder einem verringerten Niveau, um den Nutzer bei der Bewegung zu unterstützen. Das relativ geringe Niveau zugeführter Energieverlängert die möglichen Einsatzzeiten ohne Wartung.

In dem Diagram gemäß der Figur 13 sind weiterhin die aufgebrachte Leistung P sowie die verrichtete Arbeit W aufgetragen. Die motorisch zugeführte Leistung P steigt zu Beginn der Einbeugung des Knies an, nach dem "toe-off" ist ein Leistungsüberschuss vorhanden, das heißt, dass keine motorische Leistung zugeführt werden muss. Nach Erreichen des Kniewinkelmaximums wird zur Extension wieder Leistung benötigt, so dass die Leistung P wieder positiv wird.

Eine Steuerung, wie sie anhand der Figur 13 beschrieben wurde, ist nicht nur mit einer hydraulischen Antriebseinrichtung durchführbar, es ist ebenso vorgesehen, dass die Beuge- und Streckmomente über einen Elektromotor direkt aufgebracht werden, ggf. unter Zwischenschaltung eines geeigneten Getriebes. Zur Dämpfung der Bewegung am Ende kann der Motor auf Generatorbetrieb umgestellt werden, wobei die erzeugte elektrische Energie in einem Akkumulator oder Kondensator gespeichert werden kann, um sie zu gegebener Zeit abrufen zu können.

## Patentansprüche

1. Verfahren zur Steuerung einer orthetischen oder prothetischen Kniegelenkeinrichtung mit einem Oberteil (2) und einem gelenkig daran angeordneten Unterteil (3), Befestigungseinrichtungen (4, 5) zum Festlegen der Gelenkeinrichtung (1) an einem Nutzer sowie einer Antriebseinrichtung zur Beaufschlagung der Kniegelenkeinrichtung mit einem Moment, wobei vor der Einbeugung die Kniegelenkeinrichtung mit einem unterstützenden Beugemoment beaufschlagt wird, das unterhalb des Niveaus liegt, das zu einer Einbeugung führt, **dadurch gekennzeichnet, dass** das Beugemoment in einem Zeitraum unmittelbar vor der Einbeugung aufgebracht wird, der zwischen 5% und 40% der Dauer eines Gangzyklusses beträgt.

2. Verfahren nach Anspruch 1, **dadurch gekennzeichnet, dass** das Beugemoment verringert wird, wenn die Kniegelenkeinrichtung eingebeugt oder bevor die Kniegelenkeinrichtung eingebeugt wird.

3. Verfahren nach Anspruch 1 oder 2, **dadurch gekennzeichnet, dass** in Abhängigkeit von der Gehsituation ein unterschiedlich hohes Beugemoment bereitgestellt wird.

4. Verfahren nach einem der Ansprüche 1 bis 3, **dadurch gekennzeichnet, dass** in Abhängigkeit von dem Prothesenaufbau ein unterschiedlich hohes Beugemoment bereitgestellt wird.

5. Verfahren nach einem der Ansprüche 1 bis 4, **dadurch gekennzeichnet, dass** das Beugemoment bis zu einem vorbestimmten, ermittelten Beugewinkel aufgebracht wird.

6. Verfahren nach einem der Ansprüche 1 bis 5, **dadurch gekennzeichnet, dass** nach dem Einleiten der Einbeugung eine Dämpfung erfolgt oder eine Pumpe (21) im Generatorbetrieb betrieben wird.

## Claims

1. Method for controlling an orthotic or prosthetic knee joint device with an upper part (2) and a lower part (3) arranged in an articulated manner thereon, fastening devices (4, 5) for securing the joint device (1) on a user and also a drive device for applying a moment to the knee joint device, wherein a supportive flexion moment, which lies below the level that leads to a flexing, is applied to the knee joint device before the flexing, **characterized in that** the flexion moment is applied in a time period directly before the flexing, which is between 5% and 40% of the duration of a gait cycle.

2. Method according to claim 1, **characterized in that** the flexion moment is reduced when the knee joint device is flexed or before the knee joint device is flexed.

3. Method as claimed in claim 1 or 2, **characterized in that** a flexion moment of a varying degree is provided in dependence on the walking situation.

4. Method as claimed in claims 1 - 3, **characterized in that** a flexion moment of a varying degree is provided in dependence on the prosthesis set-up.

5. Method as claimed in claims 1 - 4, **characterized in that** the flexion moment is applied up to a predetermined, ascertained flexion angle.

6. Method as claimed in claims 1 - 5, **characterized in that**, after the initiation of the flexing, a damping takes place or a pump (21) is operated in generator mode.

## Revendications

1. Procédé pour la commande d'un système d'articulation orthétique ou prothétique pour le genou avec une partie supérieure (2) et une partie inférieure (3) agencée de façon articulée sur celle-ci, des moyens de fixation (4, 5) pour immobiliser le système d'articulation (1) sur un utilisateur, ainsi qu'un moyen d'entraînement pour appliquer un couple au système d'articulation, de sorte que, avant la flexion, le système d'articulation est sollicité avec un couple de flexion d'assistance, situé au-dessous du niveau qui mène à une flexion entrante, **caractérisé en ce que** le couple de flexion est épuisé dans une période temporelle immédiatement avant la flexion entrante, qui s'élève entre 5 % et 40 % de la durée d'un cycle de marche.

2. Procédé selon la revendication 1, **caractérisé en ce que** le couple de flexion est réduit quand le système d'articulation est mis en flexion entrante ou avant que le système d'articulation soit mis en flexion entrante.

3. Procédé selon la revendication 1 ou 2, **caractérisé en ce qu'**un couple de flexion d'intensité différente est fourni en fonction de la situation de marche.

4. Procédé selon l'une des revendications 1 à 3, **caractérisé en ce qu'**un couple de flexion d'intensité différente est fourni en fonction de la structure de la prothèse.

5. Procédé selon l'une des revendications 1 à 4, **caractérisé en ce que** le couple de flexion est épuisé jusqu'à un angle de flexion constaté prédéterminé.

6. Procédé selon l'une des revendications 1 à 5, **caractérisé en ce qu'**après démarrage de la flexion entrante il se produit un amortissement, ou bien une pompe (21) est amenée à fonctionner en mode générateur.
